# EUROPÄISCHE PATENTSCHRIFT

(11) **EP 0 519 292 B1**
(45) Veröffentlichungstag und Bekanntmachung des Hinweises auf die Patenterteilung: **25.10.1995**
(21) Anmeldenummer: 92109626.9
(22) Anmeldetag: 09.06.1992
(51) Int. Cl.: A61M 5/20

(54) **Spritzenartige Vorrichtung zum Dosieren von Flüssigkeiten oder Pasten**
Siringe-like device for dosing fluids or pastes
Dispositif ressemblant une seringue pour le dosage de produits liquides ou pâteux

(30) Priorität: 20.06.1991 DE 9107574 U
(43) Veröffentlichungstag der Anmeldung: 23.12.1992
(73) Patentinhaber: BAYER AG, 51368 Leverkusen (DE)
(72) Erfinder: Diederich, Reiner, Dipl.-Ing., W-5047 Wesseling (DE); Von Schuckmann, Alfred, W-4178 Kevelaer 2 (DE)

(56) Entgegenhaltungen:
- AU-A- 478 956
- DE-A- 2 106 338
- DE-A- 3 527 066
- FR-A- 2 342 744
- US-A- 3 827 601

## Beschreibung

Die Erfindung betrifft eine spritzenartige Vorrichtung zum Dosieren von Flüssigkeiten oder Pasten gemäß dem Oberbegriff von Anspruch 1. Eine derartige Vorrichtung ist aus der AU-A-478956 bekannt.

Im medizinischen, insbesondere im zahnmedizinischen, Bereich benötigt man spritzenartige Vorrichtungen, auch Applikatoren genannt, zum Dosieren von Flüssigkeiten oder dünnflüssigen Pasten.

Bei bekannten Vorrichtungen muß man die Flüssigkeit mittels des Kolbens ansaugen, um die innerhalb der Vorrichtung befindliche Vorratskammer zu füllen. Diese Handhabung ist umständlich, Flüssigkeit kann dabei verschmutzt, verschüttet oder der Behälter umgestoßen werden. Außerdem läßt sich der Behälter nicht restlos entleeren.

Es besteht die Aufgabe, die Vorrichtung der eingangs genannten Art so zu verbessern, daß das Füllen und die Handhabung einfacher vonstatten geht und der Behälter sich restlos entleeren läßt.

Die Erfindung geht von einem Stand der Technik aus (AU-A 478956), bei dem der Auslaßöffnung ein Ventil vorgeordnet ist; dem Ventil ist eine Dosierkammer mit einem darin geführten Kolben vorgeordnet, wobei durch den Kolben und seinen anschließenden Schaft ein Kanal geführt ist. Erfindungsgemäß ist das Ende dieses Schaftes in einem im Gehäuse verschiebbar geführten Verbindungsrohr abdichtend gehalten, wobei zwischen dem Kanal des Schaftes und dem Kanal des Verbindungsrohres ein Ventil angeordnet ist; am Ende des Verbindungrohres ist eine Halterung für eine abdichtend fixierbare Vorratskammer in Form eines austauschbaren Behälters angeordnet und das Verbindungsrohr ist mittels eines Hebels gegen eine sich am Gehäuse abstützende Rückstellfeder in Richtung Auslaßöffnung betätigbar.

Diese Ausbildung der Vorrichtung hat den Vorteil, daß die die Flüssigkeit enthaltenden Vorratsbehälter direkt an der Vorrichtung befestigt werden können, so daß ihr Inhalt, ohne daß es besonderer Manipulationen bedarf, nach und nach, und zwar restlos, verbraucht werden kann. Die Behälter lassen sich beispielsweise mittels Schraubgewinde an das Verbindungsrohr abdichtend anschließen, wobei dieses Schraubgewinde vorher als Halterung für die Verschlußkappe des Behälters dient. Sobald der Behälter fixiert ist, ist der Inhalt geschützt. Ein Verschütten der Flüssigkeit kann lediglich beim Fixieren des Behälters erfolgen. Wesentlich ist aber auch, daß der Behälter sich restlos entleeren läßt. Die Vorrichtung läßt sich vollständig aus Kunststoffteilen, welche im Spritzgußverfahren gefertigt wurden, herstellen und montieren. Das Ventil besteht in einfachster Weise aus einer planen Dichtscheibe, welche beim Entstehen des Unterdruckes die Austrittsöffnung des Kanals des Verbindungsrohres verschließt.

Gemäß einer ersten besonderen Ausführungsform ist am in den Behälter hineinragenden Ende des Verbindungsrohres ein mit der Atmosphäre verbundenes Belüftungsventil angeordnet.

Auf diese Weise wird der beim Dosieren sich im Behälter aufbauende Unterdruck ausgeglichen, indem Luft nachgesaugt wird. Geeignet sind ein gummielastisches Ventil oder ein federbelastetes Kugelventil.

Es ist jedoch auch möglich, den Behälter faltenbalgartig zu gestalten oder als Kartusche mit Schleppkolben auszubilden.

Diese Ausführungsformen sind insbesondere dann vorteilhaft, wenn die Flüssigkeit nicht mit Luft in Berührung kommen soll. Der Faltenbalg bzw. der Schleppkolben sorgen dann dafür, daß durch Volumenausgleich der Unterdruck aufgehoben wird.

In der Zeichnung ist die neue Vorrichtung rein schematisch dargestellt und nachstehend näher erläutert. Es zeigen:
- Fig. 1: die Vorrichtung im Längsschnitt und
- Fig. 2 und 3: abgewandelte Ausführungsformen des Behälters der Vorrichtung.

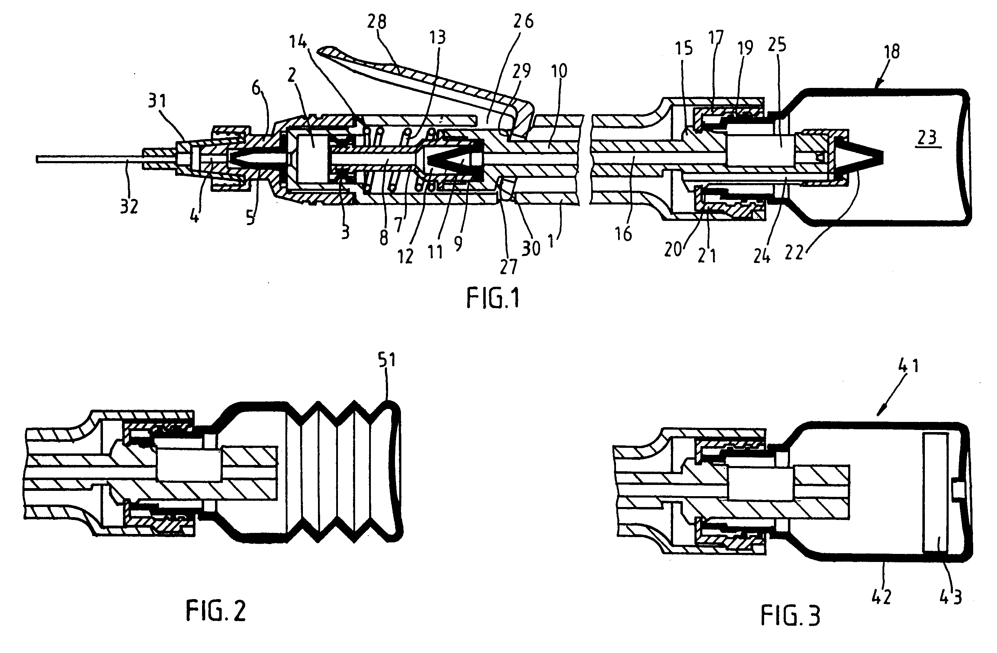

In Fig. 1 besteht die Vorrichtung aus einem Gehäuse 1, in welchem in einer Dosierkammer 2 ein Kolben 3 geführt ist. Einer Auslaßöffnung 4 vorgeordnet befindet sich ein Ventil 5 aus gummielastischem Material, welches zwischen einem Kopfstück 6 und dem Gehäuse 1 eingespannt ist. Der Kolben 3 geht in einen Schaft 7 über. Beide sind von einem axial angeordneten Kanal 8 durchzogen. Der Schaft 7 ist in einer Ausnehmung 9 eines im Gehäuse 1 verschiebbar gelagerten Verbindungsrohres 10 abdichtend eingeschraubt. In dem freibleibenden Teil der Ausnehmung 9 ist ein Ventil 11 angeordnet, welches ebenfalls aus gummielastischem Material besteht. Es ragt in eine Erweiterung 12 des Kanals 8 im Schaft 7 hinein. Der Schaft 7 ist von einer Rückstellfeder 13 umgeben, welche sich einerseits an einem Absatz 14 des Gehäuses 1 abstützt und andererseits gegen das Verbindungsrohr 10 drückt. Das Ende 15 des ebenfalls von einem Kanal 16 durchzogenen Verbindungsrohres 10 weist eine Halterung 17 auf, in welcher ein Behälter 18 mittels Gewindeverbindung 19 gehalten ist. Damit der Rand 20 des Behälters 18 abgedichtet wird, weist das Verbindungsrohr 10 an dieser Stelle einen Konusansatz 21 auf. Außerdem ist das Ende 15 des Verbindungsrohres 10 noch mit einem Belüftungsventil 22 aus gummielastischem Material versehen, welches den Innenraum 23 des Behälters 18 über einen Kanal 24 mit der Atmosphäre verbinden kann. Eine Ausnehmung 25 sichert den Fluß vom Innenraum 23 in den Kanal 24. Das Gehäuse 1 ist mit gegenüberliegenden Ausnehmungen 26 und 27 versehen, in welche ein Hebel 28 eingreift. Dieser liegt gegen Absätze 29 und 30 des Verbindungsrohres 10 an, wobei die Lage der Ausnehmungen 26, 27 und der Absätze 29, 30 im Zusammenhang mit der Geometrie des Hebels 28 so abgestimmt ist, daß beim Betätigen des Hebels 28 das Verbindungsrohr 10 gegen die Spannkraft der Rückstellfeder 13 in Richtung Auslaßöffnung 4 verschoben wird. Auf das Kopfstück 6 ist außen ein Adapter 31 mit Kanal 32 aufgesteckt.

In Fig. 2 ist der Behälter 51 faltenbalgartig ausgebildet und besteht aus einem elastischen Kunststoff.

In Fig. 3 besteht der Behälter 41 aus einer Kartusche 42 mit einem Schleppkolben 43.

Die Arbeitsweise der neuen Vorrichtung gemäß Fig. 1 ist folgende:
Zunächst wird der Behälter 18 an der Halterung 17 angebracht, indem seine Verschlußkappe entfernt und anschließend bei nach oben gehaltenem Gehäuse 1 die Gewindeverbindung 19 hergestellt wird. Sodann hält man die Vorrichtung nach unten, damit die Flüssigkeit durch die Kanäle 16 und 8 in die Dosierkammer 2 strömt. Das Ventil 5 ist dank seiner Elastizität geschlossen. Durch Betätigen des Hebels 28 wird das Verbindungsrohr 10 samt Kolben 3 in Richtung Auslaßöffnung 4 verschoben, wodurch das Ventil 11 durch den entstehenden Unterdruck geschlossen und das Ventil 5 durch den Druck gedehnt und geöffnet wird. Ein entsprechend dem Vorschub des Kolbens verdrängtes Volumen an Flüssigkeit wird nun dosiert. Beim Loslassen des Hebels 28 drückt die Rückstellfeder 13 das Verbindungsrohr 10 samt Kolben 3 wieder in ihre Ausgangsposition. Dabei schließt das Ventil 5, und das Ventil 11 öffnet durch den im Kanal 8 und in der Dosierkammer 2 entstehenden Unterdruck, so daß wieder Flüssigkeit in die Kammer 2 gesaugt wird. Dabei entsteht auch Unterdruck in dem Behälter 18, wodurch sich das elastische Belüftungsventil 22 öffnet und aus der Atmosphäre Luft über den Kanal 24 in den Innenraum 23 des Behälters 18 einsaugt. Die Vorrichtung ist nun für den nächsten Dosiervorgang bereit. Ist der Behälter 18 leer, wird er gegen einen neuen ausgetauscht.

Die Arbeitsweise der Vorrichtung gemäß der abgewandelten Ausführungsform nach Fig. 2 erfolgt wie oben beschrieben, jedoch mit dem Unterschied, daß wegen der fehlenden Belüftungsmöglichkeit der Faltenbalg des Behälters 51 den Volumenausgleich übernimmt, wodurch der Unterdruck abgebaut wird.

## Patentansprüche

1. Spritzenartige Vorrichtung zum Dosieren von Flüssigkeiten oder Pasten, bestehend aus einem Gehäuse (1) mit Kolben (3), einer Vorratskammer (18, 51, 41), die Mittel zum Ausgleich des sich beim Dosieren aufbauenden Unterdrucks aufweist und einer Auslaßöffnung (4) am Ende des Gehäuses (1), wobei der Auslaßöffnung (4) ein Ventil (5) vorgeordnet ist; dem Ventil (5) eine Dosierkammer (2) mit dem darin geführten Kolben (3) vorgeordnet ist, und durch den Kolben (3) und seinen anschließenden Schaft (7) ein Kanal (8) geführt ist; dadurch gekennzeichnet, daß das Ende dieses Schaftes (7) in einem im Gehäuse (1) verschiebbar geführten Verbindungsrohr (10) abdichtend gehalten ist, wobei zwischen dem Kanal (8) des Schaftes (7) und dem Kanal (16) des Verbindungsrohres (10) ein Ventil (11) angeordnet ist; daß an dem der Vorratskammer benachbarten Ende (15) des Verbindungsrohres (10) eine Halterung (17) für die abdichtend fixierbare Vorratskammer in Form eines austauschbaren Behälters (18, 51, 41) angeordnet ist und daß das Verbindungsrohr (10) mittels eines Hebels (28) gegen eine sich am Gehäuse (1) abstützende Rückstellfeder (13) in Richtung Auslaßöffnung (4) betätigbar ist.

2. Spritzenartige Vorrichtung nach Anspruch 1, dadurch gekennzeichnet, daß am in den Behälter (18) hineinragenden Ende (15) des Verbindungsrohres (10) ein mit der Atmosphäre verbundenes Belüftungsventil (22) angeordnet ist.

3. Spritzenartige Vorrichtung nach Anspruch 1, dadurch gekennzeichnet, daß der Behälter (51) faltenbalgartig ausgebildet ist.

4. Spritzenartige Vorrichtung nach Anspruch 1, dadurch gekennzeichnet, daß der Behälter (41) aus einer Kartusche (42) mit Schleppkolben (43) besteht.

## Claims

1. Syringe-like device for the dosing of liquids or pastes, consisting of a housing (1) with a plunger (3), a storage chamber (18, 51, 41) which has means for compensating for the partial vacuum building up during dosing, and an outlet opening (4) at the end of the housing (1), a valve (5) being arranged upstream of the outlet opening (4), a dosing chamber (2) with the plunger (3) guided therein being arranged upstream of the valve (5) and a channel (8) leading through the plunger (3) and its adjoining shaft (7), characterised in that the end of this shaft (7) is held in a leakproof manner in a connection tube (10) guided displaceably in the housing (1), a valve (11) being arranged between the channel (8) of the shaft (7) and the channel (16) of the connection tube (10); in that at that end (15) of the connection tube (10) which is adjacent to the storage chamber there is a holding arrangement (17) for the storage chamber which can be fitted in a leakproof manner in the form of an exchangeable container (18, 51, 41), and in that the connection tube (10) can be moved by means of a lever (28) in the direction of the outlet opening (4) counter to a return spring (13) bearing on the housing (1).

2. Syringe-like device according to Claim 1, characterised in that an aeration valve (22) connected to the atmosphere is arranged at the end (15) of the connection tube (10) projecting into the container (18).

3. Syringe-like device according to Claim 1, characterised in that the container 51 is designed in a bellows formation.

4. Syringe-like device according to Claim 1, characterised in that the container (41) consists of a cartridge (42) with a draw plunger (43).

## Revendications

1. Dispositif sous forme de seringue destiné au dosage de produits liquides ou pâteux comportant un boîtier (1) avec un piston (3), un réservoir (18, 51, 41) présentant un moyen pour compenser le vide naissant lors du dosage, et un orifice de sortie (4) à l'extrémité du boîtier (1), une soupape (5) précédant l'orifice de sortie (4), une chambre de dosage (2) dans laquelle est introduit un piston (3) précédant la soupape (5) et un canal (8) traversant le piston (3) et sa tige adjacente (7); caractérisé en ce que l'extrémité de cette tige (7) est maintenue étanche dans un tube de raccordement (10) guidé à coulissement dans le boîtier (1), une soupape (11) étant disposée entre le canal (8) de la tige (7) et le canal (16) du tube de raccordement (10); en ce qu'une fixation (17) pour le réservoir, fixable de manière étanche, est disposée sur l'extrémité (15) du tube de raccordement (10) proche du réservoir sous la forme d'un réservoir remplaçable (18, 51, 41) et que le tube de raccordement (10) peut être actionné au moyen d'un levier (28) contre un ressort de rappel (13) prenant appui sur le boîtier (1), en direction de l'orifice de sortie (4).

2. Dispositif sous forme de seringue selon la revendication 1, caractérisé en ce qu'une soupape d'aération (22) communiquant avec l'atmosphère est disposée sur l'extrémité (15) du tube de raccordement (10) faisant saillie à l'intérieur du réservoir (18).

3. Dispositif sous forme de seringue selon la revendication 1, caractérisé en ce que le réservoir (51) présente la forme d'un soufflet.

4. Dispositif sous forme de seringue selon la revendication 1, caractérisé en ce que le réservoir (41) comporte une cartouche (42) pourvue d'un piston d'entraînement (43).
